Europäisches Patentamt

European Patent Office

Office européen des brevets

(19)

(11) **EP 1 171 143 B1**

(12) **FASCICULE DE BREVET EUROPEEN**

(45) Date de publication et mention
de la délivrance du brevet:
**20.10.2004 Bulletin 2004/43**

(21) Numéro de dépôt: **00920831.5**

(22) Date de dépôt: **18.04.2000**

(51) Int Cl.7: **A61K 35/78**, A61K 7/48,
A61P 19/02, A61P 1/02,
A61P 17/00, A61P 29/00,
A61P 17/02, A61P 9/00

(86) Numéro de dépôt international:
**PCT/FR2000/001007**

(87) Numéro de publication internationale:
**WO 2000/062789 (26.10.2000 Gazette 2000/43)**

(54) **EXTRAIT PEPTIDIQUE DU LUPIN ET COMPOSITION PHARMACEUTIQUE OU COSMETIQUE OU NUTRACEUTIQUE COMPRENANT UN TEL EXTRAIT**

PEPTIDEXTRAKT AUS LUPINEN UND PHARMAZEUTISCHE ODER KOSMETISCHE ODER PHARMACO-NAHRUNGSMITTEL ZUSAMMENSETZUNG, DIE SO EIN EXTRAKT ENTHALTEN

PEPTIDE EXTRACT OF LUPINE AND PHARMACEUTICAL OR COSMETIC OR NUTRITIONAL COMPOSITION COMPRISING SAME

(84) Etats contractants désignés:
**AT BE CH CY DE DK ES FI FR GB GR IE IT LI LU MC NL PT SE**

(30) Priorité: **19.04.1999 FR 9904875**

(43) Date de publication de la demande:
**16.01.2002 Bulletin 2002/03**

(73) Titulaire: **Laboratoires Expanscience**
**92419 Courbevoie Cedex (FR)**

(72) Inventeurs:
• **MSIKA, Philippe**
**F-75018 Paris (FR)**
• **PICCIRILLI, Antoine**
**F-28230 Epernon (FR)**
• **PAUL, François**
**F-31400 Toulouse (FR)**

(74) Mandataire: **Ahner, Francis**
**Cabinet Régimbeau**
**20, rue de Chazelles**
**75847 Paris cedex 17 (FR)**

(56) Documents cités:
• **M. RADLOWSKI ET AL.: "SYSTEMIN-AN INDUCER OF PROTEINASE INHIBITOR SYNTHESIS CAN BE RESPONSIBLE FOR BIOLOGICAL ACTIVITY OF A LUPIN EXTRACT AGAINST INSECTS." JOURNAL OF PLANT PHYSIOLOGY., vol. 150, 1997, pages 220-223, XP000866580 FISCHER, STUTTGART., DE ISSN: 0176-1617**
• **MARK E. HINES ET AL.: "SCREENING FOR CYSTEINE PROTEINASE INHIBITOR ACTIVITY IN LEGUME SEEDS." JOURNAL OF THE SCIENCE OF FOOD AND AGRICULTURE., vol. 59, 1992, pages 555-557, XP002126982 ELSEVIER APPLIED SCIENCE PUBLISHERS. BARKING., GB ISSN: 0022-5142**

## Description

La présente invention est relative à un nouvel extrait peptidique présentant une activité anti-métalloprotéase, notamment anti-collagénase et anti-gélatinase. Elle est également relative aux compositions pharmaceutiques, cosmétiques ou nutraceutiques comprenant un tel extrait, notamment une composition pharmaceutique destinée à traiter les maladies inflammatoires, telles que l'arthrose, la parodontose, ou les ulcères ou les compositions cosmétiques destinées à combattre le vieillissement actinique ou non ou accéléré par les agressions extérieures (tabac, pollution, ...).

La composition pharmaceutique ou cosmétique ou nutraceutique est également destinée à traiter la néoangiogénèse (prolifération des vaisseaux) pathologique ou inesthétique (psoriasis, tumeurs, érythose, couperose, rosacée, traitement local aux irritants tels que l'acide rétinoïque), les défauts de cicatrisation, les brûlures ou l'attaque d'émail dentaire (Ch. M. Lapiere, Cours de biologie de la peau - COBIP INSERM U 346, Lyon 1999).

Les métalloprotéases sont une famille d'endopeptidases zinc et calcium-dépendantes qui ont la propriété combinée de dégrader les divers composants des matrices du tissu conjonctif ( Thèse de S. Charvat - Métalloprotéinases et épiderme, pages 101-113 n° 248-98, 1998, Lyon I).

Elles sont classées suivant la nature de leur substrat. La collagénase (le collagène fibrillaire : ex. MMP 1, 13, 8) ; la gélatinase (le collagène dénaturé, gélatine : ex. MMP2, MMP9) ; les stromélysines (fibronectine, protéoglycane : ex. MMP3, MMP10). Elles sont mises en jeu dans le remodelage physiologique (faible expression) ou pathologique de la matrice extracellulaire (forte induction).

Les métalloprotéases sont notamment impliquées dans le processus de cicatrisation en éliminant les tissus endommagés.

Les MMP peuvent agir de façon anarchique et conduire à des lésions importantes si leur activité n'est pas contrôlée.

Par ailleurs, on sait que les métalloprotéases sont impliquées dans certains désordres biologiques tels que les maladies inflammatoires, notamment l'arthrose, la parodontose (H. BIRKEDAL-HANSEN *et al., Critical Reviews in Oral Biology and Medicine,* **4(2)** :197 :250 (1993)) ou dans les processus de vieillissement, notamment liés à l'action du rayonnement solaire (MARTIN RIEGER ; Allured's Cosmetics & Toiletries®, vol. 114, No. 1/January 1999 ou G. J. FISHER *et al., The New England Journal of Medicine*, vol. 337, n° 20 pp. 1419-1428, *« Pathophysiology of premature skin aging induced by ultraviolet light »* et G. J. FISHER *et al., the Society for Investigative Dermatology,* Inc. 1998, pp. 61-68 *« Molecular mechanisms of photoaging and its prevention by retinoic acid : ultraviolet irradiation induces MAP kinase signal transduction cascades that induce A-1-regulated matrix metalloproteinases that degrade human skin in vivo.* ») ou dans les inflammations aiguës et chroniques (XIE *et al. ; J. Biol. Chem.* **273** : pp. 11576-11582 ; 1998) et les maladies bulleuses (nécrolyse épithéliale toxique), les pathologies à hyper prolifération cellulaire lors d'inflammation ou d'irritation, les escarres, les brûlures et les ulcères.

Il en est de même pour la prolifération des cellules endothéliales néoangiogénèses qui ont besoin dans leur phase proliférative lors de processus inflammatoires ou pathologiques (psoriasis, tumeurs) des MPP pour détruire le tissu conjonctif, afin de migrer vers d'autres territoires et de se constituer en microtubules et capillaires *(Controlling the vasculature : angiogenesis, anti-angiogenesis and vascular targeting of gene therapy* - T.P. D. FAN, R. JAGGAR et R. BICKNELL ; TiPS - February 1995, vol. 16 ; *Natural Products as angiogenesis inhibitors,* D.H. PAPER, *Planta Medical* **64** (1998) pp. 686-695 ; *Membrane-type matrix metalloproteinases in human dermal microvascular endothelial cells* : *expression and morphogenetic correlation* - V.T. CHAN *et coll.* , J.I.D. 111, pp. 1153-1159, 1998 ; *Matrix metalloproteinases in blood vessel development in human fetal skin and in cutaneous tumors* - T.V. KARELINA *et coll.* J.I. D. ; **105**, 411-417, 1995 ; *Vascular profiferation and angiogenic factors in psoriasis,* J.D. CREAMER et J.N.W.N. BARKER, *Clinical and Experimental Dermatology*, 1995,**20**, pp. 6-9).

On connaît également le rôle des inhibiteurs de métalloprotéases, notamment des collagénases, des gélatinases et des stromélysines, dans certains des traitements des maladies précitées.

L'objet de la présente invention est de proposer un nouvel inhibiteur à spectre large des métalloprotéases de type collagénase ou gélatinase permettant le traitement d'humains ou de mammifères souffrant d'une condition ou d'une maladie liée à une dégradation excessive ou pathologique du collagène ou d'une autre macroprotéine extracellulaire de soutien ou tout autre maladie liée à un excès d'expression de ces enzymes protéolytiques.

L'invention a pour objet un extrait peptidique de lupin (*lupinus*) caractérisé en ce qu'il présente une activité d'inhibition des métalloprotéases, notamment les collagénases ou les gélatinases. Comme variété de lupin, on cite notamment le genre lupin blanc doux (*lupinus albus*) tel que la variété Ares de faible teneur en alcaloïdes.

Selon une variante, cet extrait peptidique de lupin est appauvri en lipides.

Il comprend avantageusement au moins 50%, de préférence au moins 70 %, de préférence au moins 80 % de peptide.

Ces peptides sont obtenus par hydrolyse de la fraction protéique de lupin.

L'hydrolyse peut être effectuée par n'importe quel moyen approprié, notamment une hydrolyse enzymatique.

Un procédé de préparation d'un tel extrait peptidique de lupin comprend les étapes suivantes :

- préparation d'un tourteau de lupin délipidé et broyé ou d'une farine de lupin (lipidée) micronisée,
- extraction des fractions protéiques et osiques solubles ou précipitation à pH acide (4 ou 5) selon le point isoélectrique,
- éventuellement séparation de la fraction protéique,
- hydrolyse de la fraction protéique et récupération, éventuellement après filtration, de l'extrait protéique.

L'invention est également relative à l'extrait protéique susceptible d'être obtenu par le procédé précité.

De façon générale, l'invention comprend les farines de lupin lipidées, les extraits peptidiques comprenant encore les sucres.

De préférence, l'extrait protéique présente la composition en acides aminés suivante (pourcentage en poids par rapport au poids total d'acides aminés).

| Amino-acides | %/AA totaux |
|---|---|
| ASP | 11,3 |
| GLU | 23,2 |
| SER | 5,1 |
| HIS | 1,7 |
| GLY | 3,4 |
| THR | 3,2 |
| ALA | 2,8 |
| ARG | 10,3 |
| TYR | 6,1 |
| CYS-CYS | 2,4 |
| VAL | 3,8 |
| MET | 0,2 |
| PHE | 7,0 |
| ILE | 3,3 |
| LEU | 7,9 |
| LYS | 3,7 |
| PRO | 4,4 |

L'invention a également pour objet une composition pharmaceutique ou cosmétique ou nutraceutique comprenant un extrait peptidique tel que décrit précédemment, éventuellement un véhicule inerte approprié, physiologiquement acceptable.

Une telle composition pharmaceutique ou dermocosmétique et nutraceutique est destinée notamment au traitement d'humains ou de mammifères souffrant d'une condition ou d'une maladie liée à une destruction excessive de collagène et/ou une destruction excessive des tissus de soutien. Une telle composition peut être utilisée à titre préventif ou curatif

Parmi ces conditions ou maladies, on cite par exemple l'arthrose, les maladies parodontales, les maladies liées aux lésions de la peau, les maladies inflammatoires, la néangiogénèse tumorale ou pathologique (érythrose, couperose, télangectasie, rosacée, psoriasis ...), le défaut de cicatrisation, les ulcères, les brûlures, les maladies bulbeuses, et l'attaque de l'émail dentaire.

L'invention est également relative aux compositions cosmétiques pour le traitement des lésions de la peau dues au vieillissement telles que les lésions provoquées par l'action du rayonnement solaire (en anglais *photoaging*), les effets délétères intrinsèques de la peau, les effets délétères du tabac.

Les compositions pharmaceutiques, dermocosmétiques ou cosmétiques se présentent, selon une variante, sous la forme d'une formulation pour application topique. L'invention a donc pour objet une méthode de traitement cosmétique comprenant l'application d'une telle composition sur la surface cutanée d'un individu.

L'extrait peptidique selon l'invention peut également être incorporé ou formulé dans un véhicule polymérique ou un système de délivrance pour une utilisation topique ou locale, comme dans le cas du traitement d'une maladie parodontale, pour être délivré directement dans la poche parodontale.

Selon une autre variante, les compositions pharmaceutiques sont sous la forme d'une formulation pour administration orale.

Ces compositions peuvent en général être formulées sous la forme de tablettes, de capsules, de pommade.

Dans le cas des nutraceutiques ou compositions alimentaires, les formes habituellement utilisées peuvent être employées.

L'invention est maintenant illustrée par les exemples de réalisation décrits ci-après à titre illustratif

I - Préparation des extraits peptidiques de lupin

I.1. - Extrait A

- Extraction et purification des protéines de lupin

Cette étape comprend une solubilisation aqueuse de la fraction soluble à pH alcalin suivie d'une séparation des insolubles :

A partir du tourteau de lupin délipidé et broyé, l'extraction des protéines est réalisée à pH 9,0 (pH ajusté par ajout de soude) avec un ratio farine/eau égal à 1/10 (p/p). La solution est incubée sous agitation à température ambiante pendant une heure. La partie insoluble du tourteau est ensuite séparée de la partie soluble par essorage. Le gâteau obtenu est lavé. La fraction soluble contenant les protéines et les sucres solubles est diafiltrée sur un module d'ultrafiltration de seuil de coupure de 10 000 daltons afin de séparer les protéines (rétentat) des sucres solubles (ultrafiltrat).

- Production et purification de peptides par hydrolyse enzymatique :

Le rétentat d'ultrafiltration contenant les protéines est ajusté à la concentration de 100 g/l puis hydrolysé à pH 8,0 en présence d'Alcalase® (NOVO NORDISK) à 55°C pendant 3 h environ. Après hydrolyse, l'enzyme est dénaturée par hydrolyse pendant 15 min à 85°C. Dès que la solution est refroidie, elle est neutralisée par ajout d'acide chlorhydrique. Les peptides obtenus sont purifiés par diafiltration sur module d'ultrafiltration de seuil de coupure de 10 000 daltons. La solution obtenue est ensuite nanofiltrée afin de dessaler (élimination du chlorure de sodium) et de concentrer la fraction peptidique. La solution de peptides est enfin décolorée à l'aide de charbon actif 3 % (1 heure à 50 °C), le charbon étant éliminé par filtration.

- Stérilisation et conditionnement de la fraction de peptides:

Avant conditionnement, la solution est microfiltrée (0,2 μm) de façon stérile puis répartie dans des contenants stériles à la concentration de 10 % en présence de conservateurs.

I.2 - Extrait B

L'extrait peptidique B est obtenu selon le procédé décrit mis en oeuvre pour obtenir l'extrait A à la différence que l'étape de décoloration est supprimée

I.3 - Extrait C

L'extrait peptidique de lupin C est obtenu selon le procédé décrit mis en oeuvre pour obtenir l'extrait A à la différence que les étapes de purification d'ultrafiltration et de décoloration sont supprimées.

II - Analyse de l'extrait peptidique A

L'extrait sec est ensuite analysé.
Présentation :

- Aspect : poudre homogène non hygroscopique
- Couleur : blanc cassé
- Quantité: 5 g

- Composition chimique :

  - Teneur en sucres totaux (dosage à l'anthrone) :     < 1 %
  - Teneur en chlorures (Kit SIGMA réf : 955-30) :     6 %
  - Teneur en eau (100 °C, 4 h) :     8 % maximum
  - Teneur en peptides :     85 %

- caractérisation

  - pH (solution à 20 g/l):     7,06
  - Solubilité (eau osmosée) :     >100 g/l

### Tableau 1 - Composition en acides aminés de l'hydrolysat

| Amino-acides | P.M. A.A. | Conc. en mM | Conc. en mg/l | % dans poudre | %/AA totaux |
|---|---|---|---|---|---|
| ASP | 133,1 | 2,078 | 276,582 | 9,9 | 11,3 |
| GLU | 147,1 | 3,858 | 567,438 | 20,3 | 23,2 |
| SER | 105,1 | 1,196 | 125,647 | 4,5 | 5,1 |
| HIS | 155,2 | 0,270 | 41,904 | 1,5 | 1,7 |
| GLY | 75,1 | 1,114 | 83,624 | 3,0 | 3,4 |
| THR | 119,1 | 0,664 | 79,023 | 2,8 | 3,2 |
| ALA | 89,1 | 0,763 | 67,983 | 2,4 | 2,8 |
| ARG | 174,2 | 1,447 | 251,980 | 9,0 | 10,3 |
| TYR | 181,2 | 0,829 | 150,215 | 5,4 | 6,1 |
| CYS-CYS | 240,3 | 0,247 | 59,234 | 2,1 | 2,4 |
| VAL | 117,1 | 0,792 | 92,743 | 3,3 | 3,8 |
| MET | 149,2 | 0,029 | 4,327 | 0,2 | 0,2 |
| PHE | 165,2 | 1,044 | 172,469 | 6,2 | 7,0 |
| ILE | 131,2 | 0,621 | 81,410 | 2,9 | 3,3 |
| LEU | 131,2 | 1,481 | 194,307 | 6,9 | 7,9 |
| LYS | 146,2 | 0,626 | 91,448 | 3,3 | 3,7 |
| PRO | 115,1 | 0,935 | 107,619 | 3,8 | 4,4 |

2447,952

Total     87,4 %

III - Activité anti-collagénase et anti-gélatinolytique des peptides de lupin - extrait A-*in vitro*

L'activité anti-collagénase a été mesurée *in vitro* dans un modèle biochimique de type screening reposant sur l'utilisation d'une collagènase purifiée et du substrat de cette dernière, la gélatine conjuguée à la fluorescéine (kit EnzChek™ Gelatinase/Collagenase, MOLECULAR PROBES). La collagénase purifiée à partir de *Clostridium histolyticum* était fournie dans le kit EnzChek™ Gelatinase/Collagenase (MOLECULAR PROBES). Cette enzyme a une double fonctionnalité sur le collagène IV (membrane basale épiderme/ derme) et gélatine.

La DQ-gélatine purifiée à partir de peau porcine et conjuguée à la fluorescéine était fournie dans le kit EnzChek™ Gelatinase/Collagenase (MOLECULAR PROBES).

Le tampon de la réaction, constitué de 0,05 M de Tris-HCl, de 0,15 M NaCl, de 5 mM de $CaCl_2$, de 0,2 mM d'azide de sodium (pH 7,6), était fourni dans le kit EnzChek™ Gelatinase/Collagenase (MOLECULAR PROBES).

L'extrait peptidique a été solubilisé dans le tampon de la réaction. Il a été testé à 0,004 ; 0,02 ; 0,04 ; 0,2 et 0,4 % (p/v).

Les dilutions des extraits à l'essai ont été incubées avec la DQ-gélatine à 1 mg/ml et la collagénase à 0,2 UI/ml pendant 1 heure, 2 heures et 24 heures à température ambiante.

Un témoin, correspondant au mélange « collagénase + DQ-gélatine », a été incubé en parallèle.

Pour chaque condition expérimentale, des échantillons, appelés par la suite « échantillons sans enzyme », ont été incubés en présence de DQ-gélatine et en absence de collagénase.

Chaque condition expérimentale a été réalisée en triplicate.

Après 1 heure, 2 heures et 24 heures, le signal correspondant à la dégradation de la DQ-gélatine a été mesurée en fluorimétrie (Excitation : 485 nm et émission : 595 nm). Pour chaque échantillon, la valeur obtenue pour les « échantillons sans enzyme » a été soustraite.

Les résultats ont été exprimés en unité de fluorescence par échantillon et en pourcentage de variation par rapport au groupe témoin.

Les groupes de données (groupe témoin et groupes traités) ont été comparés par une analyse de la variance à un facteur (ANOVA 1, $p<0,05$), suivie par un test de Dunnett. L'effet des extraits a ainsi été comparé à celui obtenu dans le groupe témoin.

L'extrait peptidique testé de 0,004 à 0,2% (p/v), présentait une activité anti-collagénase et anti-gélatinolytique dose-dépendante. L'effet était maximum au temps 24 heures comme indiqué dans le tableau 2 ci-dessous.

Tableau 2

| Temps d'incubation = 1 heure | | | | | |
|---|---|---|---|---|---|
| Témoin | 0,004 | 0,02 | 0,04 | 0,2 | 0,4 |
| 16237 | 14161 | 11890 | 11205 | 11249 | 9434 |
| 14329 | 13561 | 11161 | 10863 | 9840 | 7544 |
| 15636 | 13965 | 11757 | 11344 | 11387 | 8878 |
| **15401** | **13896*** | **11603*** | **11137*** | **10825*** | **8619*** |
| +/- | +/- | +/- | +/- | +/- | +/- |
| **976** | **306** | **388** | **248** | **856** | **971** |
| *100 %* | *89* | *75* | *73* | *73* | *57* |
| **Temps d'incubation = 2 heures** | | | | | |
| Témoin | 0,004 0,02 0,04 0,2 0,4 | | | | |
| 24776 | 20526 | 13689 | 11000 | 7617 | 6853 |
| 22516 | 19597 | 6710 | 10406 | 6072 | 4933 |
| 23779 | 20144 | 13148 | 11349 | 7824 | 6467 |
| **23690** | **20089*** | **11182*** | **10918*** | **7171*** | **6084*** |
| +/- | +/- | +/- | +/- | +/- | +/- |
| **1133** | **467** | **3883** | **477** | **957** | **1016** |
| *100 %* | *85* | *55* | *48* | *33* | *27* |
| **Temps d'incubation = 24 heures** | | | | | |
| Témoin | 0,004 | 0,02 | 0,04 | 0,2 | 0,4 |
| 31653 | 12655 | 2583 | 2378 | 524 | 1154 |
| 29536 | 11531 | 1487 | 1442 | 484 | 467 |
| 29745 | 13008 | 2657 | 2713 | 693 | 927 |

* : moyenne significativement différente du groupe témoin ($p<0,05$).

Tableau 2   (suite)

| Temps d'incubation = 1 heure | | | | | |
|---|---|---|---|---|---|
| Temps d'incubation = 24 heures | | | | | |
| Témoin | 0,004 | 0,02 | 0,04 | 0,2 | 0,4 |
| **30311**<br>**+/-**<br>**1167**<br>*100 %* | **12398***<br>**+/-**<br>**771**<br>*41* | **2242***<br>**+/-**<br>**655**<br>*7* | **2178***<br>**+/-**<br>**659**<br>*7* | **567***<br>**+/-**<br>**111**<br>*2* | **849***<br>**+/-**<br>**350**<br>*3* |
| Les résultats sont exprimés en unité de fluorescence/échantillon.<br>En gras : moyenne et écart type | | | | | |

* : moyenne significativement différente du groupe témoin (p<0,05).

En conclusion, dans les conditions expérimentales retenues, l'extrait protéique testé entre 0,004 et 0,4 % (p/v), présentait une activité anti-gélatinase/collagénase dose-dépendante. On note notamment un excellent rapport effet/dose/temps des peptides de lupin par rapport à la collagénase aspécifique : à 24 heures par exemple, 0,04 % inhibe 93 % de l'activité gélatinolytique de la collagénase de Clostridium, à 2 heures : 52 %.

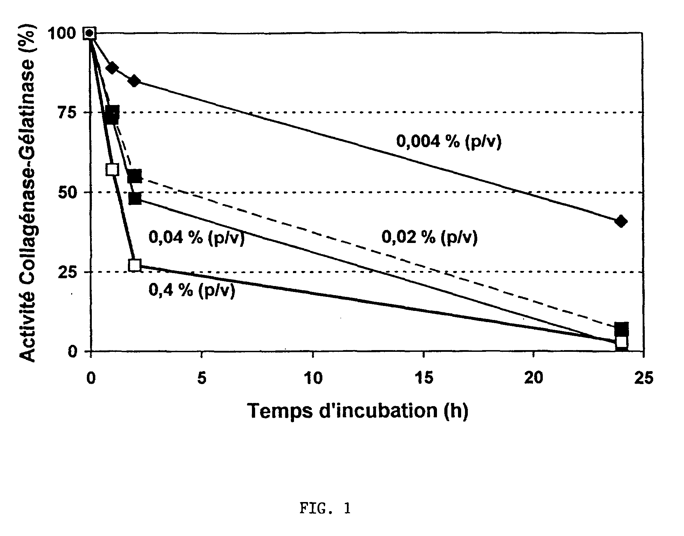

D'autres essais avec le même extrait peptidique ont été effectués à des concentrations de 0,01 ; 0,05 ; 0,1 ; 0,5 et 1 % (p/v) et les résultats sont indiqués au tableau 3 ci-dessous ainsi que sur la figure 1 annexée. Cette figure représente la cinétique d'inhibition de la collagénase aspécifique - Influence de la concentration en extrait peptidique. En ordonnée activité de la gélatinolytique (%), en abscisse temps d'incubation (h).

Tableau 3

| Temps d'incubation (h) | Concentration en extrait A (% p/v)/Activité gélatinolytique en (%) | | | | |
|---|---|---|---|---|---|
| | 0,01 | 0,05 | 0,1 | 0,5 | 1 |
| 1 | 89 | 75 | 73 | 73 | 57 |
| 2 | 85 | 55 | 48 | 33 | 27 |
| 4 | 79 | 38 | 29 | 14 | 12 |
| 6 | 69 | 27 | 21 | 8 | 9 |
| 24 | 41 | 7 | 7 | 2 | 3 |

Dans les conditions expérimentales retenues, l'extrait peptidique testé entre 0,01 et 0,5 % (p/v), présente une activité anti-gélatinase et collagénase dose dépendante et temps dépendante.

Dans tous les essais, la 1,10-phénanthroline, inhibiteur aspécifique, a été utilisée comme produit anti MMP de référence. Les résultats obtenus étaient conformes à ceux qui étaient attendus et validaient les essais.

IV - Activité anti-collagénase spécifique des peptides de lupin - extrait A- sur modèle organotypique humain

Le vieillissement cutané se caractérise, entre autre, par une modification des propriétés mécaniques cutanées de la peau, consécutive à une dégradation des fibres collagéniques du derme et d'autres macroprotéines. Cette dégradation met en jeu des collagénases endogènes (Grymes, R.A., Kronberger A. and Bauer E.A. - *Collagenases in disease* - In *«Connective Tissue Diseases of the skin* » Eds. Lapière C.M. and Krieg T., 1993, 69-85). G. Fischer et J. Voorhees *« Pathiophysiology of premature skin aging induced by ultraviolet light »* et *« Molecular mechanisms of photoaging and its prevention by retinoic acid : ultraviolet irradiation induces MAP kinase signal transduction cascades that induce A-1-regulated matrix metalloproteinases that degrade human skin in vivo»).*

Pour lutter contre les signes de vieillissement de la peau, des produits présentant une activité anti-collagénase sont envisageables.

L'activité anti-collagénase d'un produit à l'essai peut être étudiée *in vitro* dans un modèle organotypique de peau humaine. Le principe du test est le suivant : une application de collagénase purifiée sur la coupe s'accompagne d'une dégradation des fibres de collagène endogènes. Les fibres de collagène sont ensuite colorées par le trichrome de Masson. La digestion des fibres de collagène endogènes par la collagénase purifiée est évaluée qualitativement par observation morphologique et quantitativement par analyse d'images. Un produit présentant une activité anti-collagénase préservera partiellement ou totalement l'intégrité des fibres de collagène mises en présence de l'enzyme.

Les produits à l'essai ont été conservés à +4 °C jusqu'au moment de leur utilisation.

**EP 1 171 143 B1**

Trois dilutions ont été testées : 0,01 ; 0,1 et 1 % (v/v).

Le phosphoramidon, utilisé comme produit de référence, provenait de chez SIGMA.

La collagénase purifiée (type III, fraction A) provenait de chez SIGMA.

Le milieu d'incubation des coupes de peau humaine, appelé par la suite "véhicule", était le tampon Tris HCl 0,15 M, pH 7,5 contenant 0,01 M de chlorure de calcium.

Les réactifs, de qualité analytique, provenaient de chez CARLO ERBA, GIBCO ou SIGMA, sauf indication contraire.

Les coupes de peau ont été réalisées à partir d'un déchet opératoire recueilli après une plastie abdominale. Le sujet était une femme de 30 ans. Des explants de peau de 4 cm de diamètre ont été réalisés. Ils ont été déposés sur un support de liège et congelés à -80°C. Des coupes transverses de 6 µm d'épaisseur ont été réalisées avec un cryomicrotome. Elles ont été fixées sur des lames de verre et maintenues hydratées avec le véhicule pendant l'essai.

Les échantillons à l'essai ont tous été repris dans de l'éthanol avant d'être dilués dans le tampon d'essai.

- La concentration finale en éthanol a été maintenue constante et égale à 0,1 % (v/v) dans les deux plus faibles dilutions de l'extrait peptidique (0,01 et 0,1 % v/v) ;
- elle a été maintenue constante et égale à 1 % (v/v) dans la dilution la plus forte (1 %, v/v).
- Des "témoins éthanol" à 0,1 et 1 % (v/v) ont été réalisés.
- Le phosphoramidon a été repris directement dans le véhicule.

 L'extrait peptidique a été testé à 0,01 ; 0,1 et 1 % (p/v).

 Le phosphoramidon a été testé à $10^{-3}$ M.

On avait donc les échantillons suivants :

Extrait : tampon ; éthanol (0,1 %, v/v ou 1 %, v/v) ; enzyme extrait (0,01 ; 0,1 et 1 %, p/v) ;

Témoin enzyme : tampon ; éthanol (0,1 %, v/v) ; enzyme ;

Témoin éthanol (sans enzyme) : tampon ; éthanol (0,1 % ou 1 %, v/v) ;

Produit réf: tampon ; éthanol ; enzyme ; phosphoramidon $10^{-3}$ M.

Les dilutions des produits à l'essai, de l'éthanol et du produit de référence ont été déposées sur les coupes de peau, à raison de 100 µl par coupe, et pré-incubées pendant dix minutes à 37 °C. Des bandes de papier filtre (0,16 cm$^2$ de surface) imbibées de véhicule seul (témoin sans enzyme) ou contenant de la collagénase à 50 unités internationales (UI)/ml (témoin enzyme) ont ensuite été déposées sur les coupes. Les lames ont été placées en chambre humide à 37°C pendant trois heures.

Après l'incubation, les coupes ont été rincées avec le milieu d'incubation et colorées par le trichrome de Masson. L'activité de l'enzyme en absence et en présence de l'extrait, de l'éthanol ou du produit de référence a été évaluée par observation microscopique et notée selon le barème suivant :

 0 : digestion enzymatique nulle

 + : digestion enzymatique faible

 ++ : digestion enzymatique moyenne

 +++ : digestion enzymatique forte.

Des photographies des coupes ont été réalisées.

L'activité de la collagénase en absence et en présence des produits à l'essai, de l'éthanol ou du produit de référence a été évaluée par analyse d'images. L'image des coupes colorées a été digitalisée sur un écran vidéo; le logiciel d'analyse d'image (EMAGENIA 2000, BIOCOM®) a permis de calculer la surface occupée par les fibres de collagène intactes. Les résultats ont été exprimés en pourcentage de fibres de collagène intactes par champ optique.

Le pourcentage d'inhibition de l'activité collagénase des extraits à différentes concentrations de l'éthanol et du produit de référence a été calculé à l'aide des formules suivantes :

Pour le produit de référence (directement repris dans le véhicule), le pourcentage d'inhibition est calculé par la formule suivante :

$$\frac{(\% \text{ fibres collagène})\text{Produit Réf} - (\% \text{ fibres collagène})\text{Témoin enzyme}}{(\% \text{ fibres collagène})\text{Témoin éthanol} - (\% \text{ fibres collagène})\text{Témoin enzyme}} \times 100$$

Pour les extraits dilués dans le véhicule contenant 0,1 % (v/v) d'éthanol), le pourcentage d'inhibition est calculé par la formule suivante :

8

$$\frac{\text{(\% fibres collagène)Produit Réf- (\% fibres collagène)Témoin enzyme}}{\text{(\% fibres collagène)Témoin éthanol - (\% fibres collagène) Témoin enzyme}} \times 100$$

Pour les extraits dilués dans le véhicule contenant 1 % (v/v) d'éthanol), le pourcentage d'inhibition est calculé par la formule suivante :

$$\frac{\text{(\% fibres collagène)Produit Réf- (\% fibres collagène)Témoin enzyme}}{\text{(\% fibres collagène)Témoin éthanol - (\% fibres collagène)Témoin enzyme.}} \times 100$$

L'activité anti-collagénase de l'extrait à différentes concentrations a été étudiée dans un modèle organotypique de peau humaine.

En absence de collagénase (témoin véhicule), les fibres de collagène étaient intactes. En présence de collagénase (témoin enzyme), les fibres de collagène étaient presque totalement dégradées). Ce résultat était attendu et validait l'essai.

Le phosphoramidon à $10^{-3}$ M, utilisé comme produit de référence, inhibait de 16% l'activité de la collagénase. Ce résultat était attendu et finissait de valider l'essai.

L'éthanol, utilisé comme diluant intermédiaire des produits à l'essai a été testé à 0,1 et 1 % (v/v). Il n'avait pas d'effet sur la dégradation des fibres de collagène par la collagénase.

L'extrait peptidique testé à 0,01; 0,1 et 1 % (p/v) inhibait l'activité de la collagénase de 2, 24 et 65 % respectivement L'observation morphologique aboutissait au même résultat.

En conclusion, dans les conditions expérimentales retenues, l'extrait peptidique A présentait à des concentrations faibles une activité anti-collagénase importante.

Les résultants d'inhibition de l'extrait peptidique, de l'éthanol et du phosphoramidon sur la digestion des fibres de collagène dermiques par la collagénase sont réunis dans le tableau ci-dessous :

Tableau 4

| Condition expérimentale | Concentration | Digestion enzymatique (observation morphologique) | % d'inhibition de l'activité collagénase (analyse d'image) |
|---|---|---|---|
| Témoin véhicule | - | 0 | - |
| Témoin enzyme | 50 UI/ml | +++ | 0 |
| Phosphoramidon (M) | $10^{-3}$ | + | + |
| Ethanol (%, v/v) | 0,1 | +++ | 0 |
| | 1 | +++ | 0 |
| Extrait peptidique (%, v/v) | 0,01 | +++ | 2 |
| | 0,1 | ++ | 24 |
| | 1 | + | 65 |

0 : digestion enzymatique nulle

+ : digestion enzymatique faible

++ : digestion enzymatique moyenne

+++ : digestion enzymatique forte

V - Activité anti-métalloprotéase MMP-2 et MMP-9 des peptides de lupin - extraits A, B et C

La MMP-2 ou gélatinase A et la MMP-9 ou gélatinase B sont des métalloprotéases qui dégradent des composants spécifiques de la matrice extracellulaire: la MMP-2 dégrade la gélatine (=collagène dénaturé), les collagènes I, IV, VII et XI, la fibronectine, la laminine et l'élastine ; la MMP-9 dégrade la gélatine, les collagènes IV, V et XIV et l'élastine. Elles jouent un rôle important dans le « photo-aging » et dans la prolifération des cellules endothéliales.

L'activité anti-MMP-2 et anti-MMP-9 des produits à l'essai a été mesurée in *vitro* dans un modèle biochimique de type screening reposant sur l'utilisation d'une MMP-2 humaine purifiée et d'une MMP-9 humaine recombinante et du substrat de ces dernières, la gélatine conjuguée à la fluorescéine (kit EnzChek™ Gelatinase/Collagenase, MOLECULAR PROBES).

La MMP-2 purifiée à partir de fibrosarcome humain provenait de chez BOEHRINGER MANNHEIM.

La MMP-9 humaine recombinante provenait de chez R&D SYSTEMS.

La DQ-gélatine purifiée à partir de peau porcine et conjuguée à la fluorescéine était fournie dans le kit EnzChek™ Gelatinase/Collagenase (MOLECULAR PROBES).

Le tampon de réaction pour l'étude de l'activité de la MMP-2 (TpR1) était constitué de 50 mM de Tris-HCl, de 0,05% (p/v) de Triton X100 et de 5 mM de CaCl$_2$, pH 7,5.

Le tampon de réaction pour l'étude de l'activité de la MMP-9 (TpR2) était constitué de 50 mM de Tris-HCl, de 0,05% (p/v) de Brij 35 et de 5 mM de CaCl$_2$, pH 7,4.

Préparation des produits à l'essai et du produit de référence

La 1,10-phénanthroline a été solubilisée dans les tampons de réaction TpR1 et TpR2. Elle a été testée à 8 et 80 μg/ml.

Les extraits peptidiques A, B et C ont été solubilisés dans les tampons de réaction TpR1 et TpR2. Ils ont été testés à 0,01; 0,1 et 1% (p/v).

MMP-2

Avant son utilisation, la MMP-2 a été activée par une incubation de 30 minutes à 37°C en présence de APMA dilué à 2,5 mM dans le tampon TpR1.

Les dilutions des produits à l'essai ou du produit de référence ont été incubées avec la DQ-gélatine, diluée à 25 μg/ml, et la MMP-2 activée, diluée à 1,25 μg/ml, pendant 24 heures à 37°C.

Un témoin, correspondant au mélange « MMP-2 + DQ-gélatine », a été incubé en parallèle.

Pour chaque condition expérimentale, des échantillons, appelés par la suite « échantillons sans enzyme », ont été incubés en présence de DQ-gélatine et en absence de MMP-2 activée. Ces échantillons permettaient de mesurer l'interférence des produits à l'essai avec la méthode d'évaluation des effets (fluorimétrie).

Chaque condition expérimentale a été réalisée en triplicate.

MMP-9

Les dilutions des produits à l'essai ou du produit de référence ont été incubées avec la DQ-gélatine, diluée à 25 μ g/ml, et la MMP-9, diluée à 0,25 μg/ml, pendant 24 heures à 37°C.

Un témoin, correspondant au mélange « MMP-9 + DQ-gélatin », a été incubé en parallèle.

Pour chaque condition expérimentale, des échantillons, appelés par la suite « échantillons sans enzyme », ont été incubés en présence de DQ-gélatine et en absence de MMP-9. Ces échantillons permettaient de mesurer l'interférence des produits à l'essai avec la méthode d'évaluation des effets (fluorimétrie).

Chaque condition expérimentale a été réalisée en triplicate.

Après 24 heures, le signal correspondant à la dégradation de la DQ-gélatine a été mesurée en fluorimétrie (Excitation : 485 nm et émission : 595 nm). Pour chaque échantillon, la valeur obtenue pour les échantillons sans enzyme' a été soustraite.

Les résultats ont été exprimés en unité de fluorescence par échantillon et en pourcentage de variation par rapport au groupe témoin.

Les groupes de données (groupe témoin et groupes traités) ont été comparés par une analyse de la variance à un facteur (ANOVA 1, $p < 0,05$), suivie par un test de Dunnett.

Les résultats sont indiqués ci-dessous :

V.1 - Activité anti-MMP-2

| Extrait peptidique | Témoin | Activité MMP-2 (en %) (1) / Concentration (% v/v) en solution d'extrait peptidique à 10 % en poids | | |
|---|---|---|---|---|
| | | 0,01 | 0,1 | 1 |
| C | 100 | 119 | 111 | 43 |
| A | 100 | 152 | 151 | 68 |
| B | 100 | 110 | 98 | 77 |

(1)Activité exprimée par rapport au groupe témoin en absence d'inhibiteur de la MMP-2

Conclusion

- La solution à 10 % en poids d'extrait de lupin C, testée à 0,01 et 0,1 % (v/v) ne présente pas d'activité anti-MMP-2. Testée à 1 % (v/v), elle inhibe la MMP-2 de 57%.
- La solution à 10 % en poids d'extrait de lupin A, testée à 0,01 à 0,1 % (v/v) ne présente pas d'activité anti-MMP-2. Testée à 1 % (v/v), elle inhibe la MMP-2 de 32 %.
- la solution à 10 % en poids d'extrait B, testée à 1 % inhibe à 23 % MMP-2.
- La phénanthroline, testée à 8 et 80 $\mu$g/ml, inhibe de 32 et 73 % respectivement l'activité de la MMP-2. Ce résultat qui était attendu valide l'essai.

V.2 - Activité anti-MMP-9

| Extrait peptidique | Témoin | Activité MMP-9 (en %) (1) / Concentration (% v/v) en solution d'extrait peptidique à 10 % en poids | | |
|---|---|---|---|---|
| | | 0,01 | 0,1 | 1 |
| A | 100 | 143 | 143 | 61 |
| B | 100 | 146 | 129 | 27 |

(1)Activité exprimée par rapport au groupe témoin en absence d'inhibiteur de la MMP-9

Conclusion:

- La solution à 10 % en poids d'extrait de lupin A, testée à 0.01 et 0.1 % (v/v) ne présente pas d'activité anti-MMP-9. Testée à 1 % (v/v), elle inhibe la MMP-9 de 39 %.
- La solution à 10 % en poids d'extrait de lupin B, testée à 0.01; 0.1 % (v/v) ne présente pas d'activité anti-MMP-9. Testée à 1 % (v/v), elle inhibe la MMP-9 de 73%.

La phénanthroline, testée à 8 et 80 $\mu$g/ml, inhibe de 80 et 76 % respectivement l'activité de la MMP-9. Ce résultat qui était attendu valide l'essai.

VI - Evaluation de l'effet des peptides de lupin sur la quantité de MMP-1-9 et -3 dans des fibroblastes humains irradiés par des rayons UVA.

L'étude a été réalisée sur des fibroblastes dermiques humains en culture monocouche. Les cellules ont été pré-incubées pendant 1 heure à 37°C en présence des produits de référence et du produit à l'essai. Puis, les cellules ont été irradiées par une dose unique de 10 J/cm$^2$ en UVA, en présence du produit à tester et des produits de référence.
Immédiatement après l'irradiation, les cellules ont été incubées pendant 48h à 37°C, toujours en présence du produit à tester et des produits de référence.
Le dosage des différentes MMPs est effectué dans les milieux de culture au moyen de kits ELISA spécifiques (Amersham).

Produits de référence et produit à l'essai.

La 1,10-phenantroline, testée à 80 $\mu$g/ml, inhibiteur non spécifique des MMPs, a été utilisé comme produit de référence.
Le mélange acide rétinoïque 10 $\mu$M + EGF 10 ng/ml a été utilisé pour ses capacités d'induction du TIMP1 (TIMP1, *Tissue Inhibitor* of MMP, inhibiteur physiologique des MMPs).
Une solution mère contenant 10% (p/v) de peptides de lupin a été préparée dans de l'eau désionisée. A partir de cette solution, des dilutions ont été réalisées dans le milieu de culture des fibroblastes. L'extrait peptidique de lupin a été testé à 0,5 ; 1 et 2% (v/v).
Des cultures témoins, irradiées et non irradiées, ont été incubées en parallèle, en l'absence des produits de référence et du produit à l'essai.

Traitement des données.

Les groupes de données (groupe témoin et groupes traités) ont été comparés par une analyse de variance à un facteur (ANOVA 1, p<0,05), suivie par un test de Dunnett. Les effets des produits de référence et du produit à l'essai ont ainsi été comparés à celui obtenu dans le groupe « cellules irradiées ».

Résultats.

Ils sont rassemblés dans le tableau ci-dessous et les résultats sont exprimés en % par rapport au groupe « cellules irradiées ».

| | | | Production de MMP (en %) | | |
|---|---|---|---|---|---|
| | Cellules témoins | Cellules irradiées | Cellules irradiées + Peptides de lupin (%, v/v) | | |
| | | | 0,5 | 1 | 2 |
| MMP-1 | 24 | 100 | 4 | 3 | 3 |
| MMP-9 | 41 | 100 | 17 | 2 | 0 |
| MMP-3 | 88 | 100 | 3 | 0 | 2 |

Conclusions.

La 1,10-phenantroline, testée à 80 µg/ml, inhibait de 99% la sécrétion de MMP-1, de 92% la sécrétion de MMP-9 et de 97% la sécrétion de MMP-3 par les fibroblastes irradiés.

Le mélange acide rétinoïque 10 µM + EGF 10 ng/ml inhibait de 58% la sécrétion de MMP-1, de 67% la sécrétion de MMP-9 et de 44% la sécrétion de MMP-3 par les fibroblastes irradiés.

Ces résultats étaient attendus et validaient la réactivité du système d'essai.

L'irradiation à la dose de 10 J/cm$^2$ augmentait d'un facteur 4,10; 2,42 et 1,13 les quantités respectives de MMP-1, -9 et -3 sécrétées par les fibroblastes. Ces résultats étaient attendus et validaient le système d'essai en ce qui concerne l'induction des MMP-1, -9 et -3 par les UVA.

L'extrait peptidique de lupin, testé à 0,5, 1 et 2% (v/v), diminuait respectivement de 96, 97 et 97% la quantité de MMP-1 sécrétée par les fibroblastes (p<0,05).

L'extrait peptidique de lupin, testé à 0,5, 1 et 2%, diminuait de 83, 98 et 100% respectivement la quantité de MMP-9 sécrétée par les fibroblastes (p<0,05).

L'extrait peptidique de lupin, testé à 0,5, 1 et 2%, diminuait de 97, 100 et 98% respectivement la quantité de MMP-3 sécrétée par les fibroblastes (p<0,05).

Ainsi, dans les conditions expérimentales retenues, l'extrait peptidique de lupin présente des propriétés inhibitrices importantes vis-à-vis de la production des MMP-1, -9 et -3 par des fibroblastes dermiques humains irradiés aux UVA.

VII - Exemples de formule pour un usage topique:

Les pourcentages sont exprimés en poids total de la composition. L'extrait peptidique de lupin est utilisé sous forme d'une solution aqueuse à 10% en poids selon l'invention ou d'une poudre lyophilisée appelée extrait peptidique forme poudre.

1. Crème anti-rougeurs pour peaux normales

| | |
|---|---|
| Eau q.s.p. | 100,000 |
| Tétraoctanoate de pentaérythrityle | 15,0 à 5,0 |
| Stéarate de glycéryle | 10,0 à 2,0 |

(suite)

| | |
|---|---|
| Néopentanoate d'isodécyle | 10,0 à 2,0 |
| Propylène glycol | 1,0 à 3,0 |
| Dextrine | 1,0 à 3,0 |
| Cyclométhicone | 1,0 à 3,0 |
| Extrait de soja (glycine de soja) | 0,1 à 10,0 |
| Extrait peptidique de lupin (solution aqueuse à 10%) | 0,1 à 10,0 |
| Dioxyde de titane | 1,0 à 3,0 |
| Cire de Candelilla (Euphorbia Cerifora) | 1,0 à 3,0 |
| Amidon de riz (Oriza Sativa) | 1,0 à 3,0 |
| Huile insaponifiable de soja (Glycine Soja) | 0,01 à 10,0 |
| Triglycérides caprylique/caprique | 0,5 à 5,0 |
| PEG-100 Stéarate | 0,5 à 5,0 |
| Extrait de Sophora Japonica | 0,1 à 10,00 |
| Acide stéarique | 0,5 à 1,0 |
| Tocophéryl Acétate | 0,1 à 1,0 |
| Phénoxyéthanol | 0,1 à 1,0 |
| CI 77891 | 0,1 à 1,0 |
| Gomme de Xanthane | 0,1 à 0,5 |
| Diméthiconol | 0,1 à 0,5 |
| Polyacrylamide | 0,1 à 0,5 |
| Mica | 0,1 à 0,5 |
| Cétéareth-20 | 0,1 à 0,5 |
| Chlorphénésine | 0,1 à 0,5 |
| Carbomère | 0,1 à 0,5 |
| Octyl palmitate | 0,1 à 0,5 |
| Trométhamine | 0,1 à 0,5 |
| Cire d'abeilles (Beeswax) | 0,1 à 0,5 |
| C13-14 Isoparaffine | 0,1 à 0,5 |
| DEA Cétyl Phosphate | 0,1 à 0,5 |
| Cétyl Alcool | 0,1 à 0,5 |
| Glucose | 0,1 à 0,5 |
| Fragrance | 0,1 à 0,5 |
| Dissodium EDTA | 0,1 à 0,5 |

2. Crème préventive anti-âge

| | % |
|---|---|
| Eau                         q.s.p. | 100,00000 |
| Tétraoctanoate de pentaérythrityle | 3,0 à 15,0 |
| Néopentanoate d'isodécyle | 3,0 à 15,0 |
| Squalane | 1,0 à 10,0 |
| Dextrine | 1,0 à 10,0 |
| Cyclométhicone | 1,0 à 10,0 |
| Alcool cétéaryle | 1,0 à 10,0 |
| Extrait peptidique de lupin (solution aqueuse à 10%) | 0,1 à 10,0 |
| Ascorbyle glucoside | 0,1 à 10,0 |
| Glycérine | 1,0 à 10,0 |
| Laureth-23 | 1,0 à 10,0 |

(suite)

|  | % |
|---|---|
| Myristyl myristate | 1,0 à 10,0 |
| Cyclopentasiloxane | 1,0 à 10,0 |
| Nylon-6 | 1,0 à 10,0 |
| Avocado Furane | 0,01 à 10,0 |
| Phénoxyéthanol | 0,1 à 1,0 |
| Cétéaryle Glucoside | 0,1 à 1,0 |
| Fragrance | 0,1 à 1,0 |
| Cire d'abeilles (Beeswax) | 0,1 à 1,0 |
| Méthylparabène | 0,1 à 0,5 |
| Citrate de sodium | 0,1 à 0,5 |
| Diméthiconol | 0,1 à 0,5 |
| Stéarate de glycéryle | 0,1 à 0,5 |
| Disodium EDTA | 0,1 à 0,5 |
| Propylparabène | 0,1 à 0,5 |
| Hydroxyde de sodium | 0,1 à 0,5 |
| Acrylates/C10-30 alkyle acrylates crosspolymères | 0,1 à 0,5 |
| Gomme de Xanthane | 0,1 à 0,5 |
| Glucose | 0,1 à 0,5 |

3. Crème anti-âge pour peaux matures

| Eau                    q.s.p | 100,0000 |
|---|---|
| Tétraoctanoate de pentraérythrityle | 1,0 à 10,0 |
| Neopentanoate d'isodécyle | 1,0 à 10,0 |
| Glycérides de coco hydrogénés | 1,0 à 10,0 |
| Huile de graine de Simmondsia Chinensis (Jojoba) | 1,0 à 10,0 |
| Squalane | 1,0 à 10,0 |
| Glycérine | 1,0 à 10,0 |
| Cyclométhicone | 1,0 à 5,0 |
| Cétéaryle Alcool | 1,0 à 5,0 |
| Myristyl Myristate | 1,0 à 5,0 |
| Laureth-23 | 1,0 à 5,0 |
| Silice | 1,0 à 5,0 |
| Extrait peptidique de lupin (solution aqueuse à 10%) | 0,1 à 10,0 |
| Gomme de sclérotium | 0,1 à 1,0 |
| Avocado Furane | 0,01 à 10,0 |
| Acide salicylique | 0,1 à 10,0 |
| Cire d'abeilles (Beeswax) | 0,1 à 10,0 |
| Polyacrylamide | 0,1 à 1,0 |
| Phénoxyéthanol | 0,1 à 1,0 |
| Glycéryl Stéarate | 0,1 à 1,0 |
| Rétinol Palmitate | 0,01 à 5,0 |
| Cétéaryl Glucoside | 0,01 à 5,0 |
| Nylon-6 | 0,01 à 5,0 |
| Dioxyde de titane | 0,01 à 5,0 |
| Fragrance | 0,1 à 5,0 |
| Acétate de tocophéryl | 0,1 à 5,0 |

(suite)

| | |
|---|---|
| Sorbate de potassium | 0,1 à 5,0 |
| Méthylparabène | 0,1 à 5,0 |
| C13-14 Isoparaffine | 0,1 à 5,0 |
| CI 77891 | 0,1 à 5,0 |
| Diméthiconol | 0,1 à 5,0 |
| Propylparabène | 0,1 à 5,0 |
| Hydroxyde de sodium | 0,1 à 5,0 |
| Laureth-7 | 0,1 à 5,0 |
| Cétéaryl Alcool | 0,1 à 5,0 |
| Cétyl Palmitate | 0,1 à 5,0 |
| Cocoglycérides | 0,1 à 5,0 |
| Disodium EDTA | 0,1 à 0,5 |
| CI 77491 | 0,1 à 0,5 |
| Acide citrique | 0,1 à 0,5 |

4. Crème anti-rougeurs pour peaux sèches à très sèches

| | |
|---|---|
| Eau                              q.s.p. | 100,00000 |
| Pétrolatum | 1,0 à 10,0 |
| Glycérides de coco hydrogénés | 1,0 à 10,0 |
| Néopentanoate d'isodécyle | 1,0 à 10,0 |
| Huile de Simmondsia Chinensis (Jojoba) | 1,0 à 2,0 |
| Butylène Glycol | 1,0 à 5,0 |
| Cétéaryl Alcool | 1,0 à 5,0 |
| Glycérine | 1,0 à 10,0 |
| Squalane | 1,0 à 10,0 |
| Extrait peptidique de lupin (solution aqueuse à 10%) | 0,1 à 10,0 |
| Laureth-23 | 1,0 à 10,0 |
| Dioxyde de titane | 1,0 à 10,0 |
| Huile insaponifiable de glycine Soja (Soybean) | 0,1 à 10,0 |
| Triglycérides caprylique/caprique | 1,0 à 5,0 |
| Phénoxyéthanol | 0,1 à 1,0 |
| Cétéaryl Glucoside | 0,1 à 1,0 |
| Extrait de graines de soja | 0,1 à 10,0 |
| Fragrance | 0,1 à 1,0 |
| Sophora Japonica | 0,1 à 10,0 |
| Acétate de tocophéryle | 0,1 à 1,0 |
| Cire de Candelilla | 0,1 à 1,0 |
| CI 77891 | 0,1 à 0,5 |
| Méthylparabène | 0,1 à 0,5 |
| Mica | 0,1 à 0,5 |
| Propylparabène | 0,1 à 0,5 |
| Ethylhéxyl palmitate | 0,1 à 0,5 |
| Chlorphénésine | 0,1 à 0,3 |
| Acrylates/C10-30 alkyl acrylates crosspolymères | 0,1 à 0,3 |
| Gomme de xanthane | 0,1 à 0,3 |
| Disodium EDTA | 0,1 à 0,3 |
| Hydroxyde de sodium | 0,1 à 0,3 |

VIII - Exemples de solutions de lavage buccal

Les pourcentages sont exprimés en poids total de la composition. L'extrait peptidique de lupin est utilisé sous forme d'une solution aqueuse à 10% en poids selon l'invention ou d'une poudre lyophilisée appelée extrait peptidique forme poudre.

1. Extrait peptidique de lupin solution aqueuse à 10% + antiseptique (Triclosan) + antiplaque (Gantrez S97BF®)

| | |
|---|---|
| Extrait Peptidique de Lupin | 2 |
| Alcool éthylique | 10 |
| Glycérine | 10 |
| Huile de ricin hydrogénée, Ethoxylée à 40 moles EO (Crémophor co410) | 0,5 |
| Poly(Méthyle vinyle éther/Acide Maléïque (Gantrez S97BF®) | 0,2 |
| Soude | 0,15 |
| Fluorure de sodium | 0,05 |
| Arôme Cannelle-Menthe | 0,1 |
| Triclosan | 0,03 |
| Chlorure de zinc | 0,01 |
| Saccharine sodique | 0,01 |
| Colorant C.I. 16255 (E 124) | 0,0025 |
| Eau purifiée                QSP | 100 |

2. Extrait peptidique de lupin solution aqueuse à 10% + antiseptique

| | |
|---|---|
| Extrait Peptidique de Lupin | 2 |
| Alcool éthylique | 10 |
| Glycérine | 10 |
| Huile de ricin hydrogénée, Ethoxylée à 40 moles EO (Crémophor co410) | 0,3 |
| Fluorure de sodium | 0,05 |
| Arôme Cannelle-Menthe | 0,1 |
| Triclosan | 0,03 |
| Chlorure de zinc | 0,01 |
| Saccharine sodique | 0,01 |
| Colorant C.I. 16255 (E 124) | 0,0025 |
| Eau purifiée                QSP | 100 |

3. Extrait peptidique de lupin forme poudre + antiseptique (Chlorure de cétylpiridinium)

| | |
|---|---|
| Extrait Peptidique de Lupin | 1 |
| Alcool éthylique | 10 |
| Glycérine | 8 |
| Huile de ricin hydrogénée, Ethoxylée à 40 moles EO (Crémophor co410) | 0,1 |
| Fluorure de sodium | 0,05 |
| Arôme Cannelle-Menthe | 0,1 |
| Chlorure de cetylpyridinium | 0,05 |
| Chlorure de zinc | 0,01 |
| Saccharine sodique | 0,01 |

(suite)

| Colorant C.I. 16255 (E 124) | | 0,0025 |
|---|---|---|
| Eau purifiée | QSP | 100 |

IX - Exemples de pâtes dentifrice

Les pourcentages sont exprimés en poids total de la composition. L'extrait peptidique de lupin est utilisé sous forme d'une solution aqueuse à 10% en poids selon l'invention ou d'une poudre lyophilisée appelée extrait peptidique forme poudre.

1. Extrait peptidique de lupin forme poudre + fluorures

| Extrait peptidique de Lupin | | 1 |
|---|---|---|
| Monofuorophosphate de sodium | | 0,75 |
| Fluorure de Sodium | | 0,10 |
| Sorbitol à 70% | | 35 |
| Silice synthétique à fort pouvoir abrasif | | 13 |
| Silice synthétique à faible pouvoir abrasif | | 5 |
| Carboxyméthylcellulose sodique | | 1,6 |
| Lauryl sulfate de sodium | | 1 |
| Arôme mentholé | | 0,85 |
| Oxyde de titane | | 0,5 |
| Lessive de soude | | 0,5 |
| Cyclamate de sodium | | 0,3 |
| Menthol | | 0,15 |
| Saccharine sodique | | 0,07 |
| Eau purifiée | QSP | 100 |

2. Extrait peptidique de Lupin solution aqueuse à 10% + fluorures

| Extrait peptidique de Lupin | | 2 |
|---|---|---|
| Monofuorophosphate de sodium | | 0,75 |
| Fluorure de Sodium | | 0,10 |
| Sorbitol à 70% | | 35 |
| Silice synthétique à fort pouvoir abrasif | | 13 |
| Silice synthétique à faible pouvoir abrasif | | 5 |
| Carboxyméthylcellulose sodique | | 1,6 |
| Lauryl sulfate de sodium | | 1 |
| Arôme mentholé | | 0,85 |
| Oxyde de titane | | 0,5 |
| Lessive de soude | | 0,5 |
| Cyclamate de sodium | | 0,3 |
| Menthol | | 0,15 |
| Saccharine sodique | | 0,07 |
| Eau purifiée | QSP | 100 |

**Revendications**

1.  Composition comprenant un extrait peptidique de lupin (*lupinus*) et éventuellement un véhicule inerte pour son utilisation comme médicament.

2.  Composition selon la revendication 1, pour le traitement d'humains ou de mammifères souffrant d'une maladie liée à une destruction excessive de collagène ou à une destruction excessive des macroprotéines de soutien par des métalloprotéases.

3.  Composition selon la revendication 2, pour le traitement de l'arthrose, de maladies parodontales, de lésions de la peau, de maladies inflammatoires, de maladies liées au défaut de cicatrisation, de maladies liées à l'attaque de l'émail des dents, ou de maladies liées à l'angiogénèse tumorale ou pathologique.

4.  Composition selon l'une quelconque des revendications 1 à 3, pour une application topique externe ou pour une administration orale.

5.  Composition selon l'une quelconque des revendications précédentes, **caractérisée en ce que** l'extrait peptidique de lupin comprend au moins 50 %, de préférence au moins 70 %, de préférence au moins 80 % de peptides.

6.  Composition selon l'une quelconque des revendications précédentes, **caractérisée en ce que** les peptides ont un poids moléculaire inférieur à 10 000 daltons.

7.  Composition selon l'une quelconque des revendications précédentes, **caractérisée en ce que** l'extrait peptidique de lupin présente la composition en acides aminés suivantes (en pourcentage en poids par rapport au poids total d'acides aminés) :

| Amino-acides | %/AA totaux |
|---|---|
| ASP | 11,3 |
| GLU | 23,2 |
| SER | 5,1 |
| HIS | 1,7 |
| GLY | 3,4 |
| THR | 3,2 |
| ALA | 2,8 |
| ARG | 10,3 |
| TYR | 6,1 |
| CYS-CYS | 2,4 |
| VAL | 3,8 |
| MET | 0,2 |
| PHE | 7,0 |
| ILE | 3,3 |
| LEU | 7,9 |
| LYS | 3,7 |
| PRO | 4,4 |

8.  Composition selon l'une quelconque des revendications précédentes, **caractérisée en ce que** l'extrait peptidique de lupin est susceptible d'être obtenu par un procédé comprenant les étapes suivantes :

    -   préparation d'un tourteau de lupin délipidé et broyé ou d'une farine de lupin (lipidée), micronisée,
    -   extraction des fractions protéiques et osiques solubles, ou précipitation des protéines au point isolélectrique,

- éventuellement séparation de la fraction protéique,
- hydrolyse de la fraction protéique et récupération éventuellement, après filtration, de l'extrait protéique.

**9.** Composition selon l'une quelconque des revendications précédentes, **caractérisée en ce que** l'extrait peptidique de lupin est obtenu par une hydrolyse de la fraction protéique du lupin.

**10.** Composition selon la revendication 9, **caractérisée en ce que** l'hydrolyse de la fraction protéique du lupin est une hydrolyse enzymatique.

**11.** Extrait peptidique de lupin, **caractérisé en ce qu'**il est obtenu par une hydrolyse de la fraction protéique du lupin.

**12.** Extrait selon la revendication 11, **caractérisé en ce que** l'hydrolyse de la fraction protéique du lupin est une hydrolyse enzymatique.

**13.** Composition pharmaceutique, cosmétique ou nutraceutique comprenant un extrait tel que défini à l'une quelconque des revendications 11 et 12.

**14.** Utilisation d'un extrait peptidique de lupin pour la préparation d'une composition pharmaceutique, cosmétique ou nutraceutique destinée au traitement d'humains ou de mammifères souffrant d'une maladie ou d'une condition liée à une destruction excessive de collagène ou à une destruction excessive des macroprotéines de soutien par des métalloprotéases.

**15.** Utilisation selon la revendication 14 pour le traitement de lésions de la peau, notamment de lésions dues au vieillissement intrinsèque de la peau, au vieillissement sous l'action du rayonnement solaire, ou aux effets délétères du tabac, de la pollution, ou du stress.

**Claims**

**1.** Composition comprising a peptide extract of lupin (*lupinus*), and, optionally, an inert vehicle, for its use as a medicinal product.

**2.** Composition according to Claim 1, for treating humans or mammals suffering from a disease linked to excessive destruction of collagen or to excessive destruction of support macroproteins, by metalloproteases.

**3.** Composition according to Claim 2, for treating arthrosis, periodontal diseases, skin lesions, inflammatory diseases, diseases linked to cicatrization deficiency, diseases linked to the attack of tooth enamel or diseases linked to tumor-related or pathological angiogenesis.

**4.** Composition according to any one of Claims 1 to 3, for external topical application or for oral administration.

**5.** Composition according to any one of the preceding claims, **characterized in that** the peptide extract of lupin comprises at least 50%, preferably at least 70%, preferably at least 80% of peptides.

**6.** Composition according to any one of the preceding claims, **characterized in that** the peptides have a molecular weight of less than 10 000 Daltons.

**7.** Composition according to any one of the preceding claims, **characterized in that** the peptide extract of lupin has the following amino acid composition (as percentage by weight relative to the total weight of amino acids):

| Amino acids | %/total AAs |
| --- | --- |
| ASP | 11.3 |
| GLU | 23.2 |
| SER | 5.1 |
| HIS | 1.7 |

(continued)

| Amino acids | %/total AAs |
|---|---|
| GLY | 3.4 |
| THR | 3.2 |
| ALA | 2.8 |
| ARG | 10.3 |
| TYR | 6.1 |
| CYS-CYS | 2.4 |
| VAL | 3.8 |
| MET | 0.2 |
| PHE | 7.0 |
| ILE | 3.3 |
| LEU | 7.9 |
| LYS | 3.7 |
| PRO | 4.4 |

8. Composition according to any one of the preceding claims, **characterized in that** the peptide extract of lupin can be obtained using a process comprising the following steps:

- preparing a lipid-free, ground lupin meal or a micronized lupin flour (containing lipid),
- extracting the soluble protein and saccharide fractions or precipitating the proteins at the isoelectric point,
- optionally separating the protein fraction,
- hydrolysing the protein fraction and recovering, optionally after filtration, the protein extract.

9. Composition according to any one of the preceding claims, **characterized in that** the peptide extract of lupin is obtained by hydrolysis of the protein fraction of lupin.

10. Composition according to Claim 9, **characterized in that** the hydrolysis of the protein fraction of lupin is enzymatic hydrolysis.

11. Peptide extract of lupin, **characterized in that** it is obtained by hydrolysis of the protein fraction of lupin.

12. Extract according to Claim 11, **characterized in that** the hydrolysis of the protein fraction of lupin is enzymatic hydrolysis.

13. Pharmaceutical , cosmetic or nutraceutical composition comprising an extract as defined in either one of Claims 11 and 12.

14. Use of a peptide extract of lupin, for preparing a pharmaceutical, cosmetic or nutraceutical composition intended for treating humans or mammals suffering from a disease or condition linked to excessive destruction of collagen or to excessive destruction of support macroproteins by metalloproteases.

15. Use according to Claim 14, for treating skin lesions, in particular skin lesions due to the intrinsic aging of the skin, to aging under the action of solar radiation, or to the deleterious effects of tobacco, of pollution or of stress.

**Patentansprüche**

1. Zusammensetzung, umfassend einen peptidischen Extrakt von Lupine (*Lupinus*) und gegebenenfalls einen inerten Träger, für deren Verwendung als Arzneimittel.

2. Zusammensetzung nach Anspruch 1 für die Behandlung von Menschen oder von Säugetieren, die unter einer Erkrankung leiden, die mit einer übermäßigen Zerstörung von Kollagen oder mit einer übermäßigen Zerstörung der Stütz-Makroproteine durch Metalloproteasen verbunden ist.

3. Zusammensetzung nach Anspruch 2 für die Behandlung von Arthrose, von parodontalen Erkrankungen, von Schädigungen der Haut, von Entzündungskrankheiten, von Erkrankungen, die mit einer fehlerhaften Narbenbildung verbunden sind, von Erkrankungen, die mit einem Angriff des Zahnschmelzes verbunden sind, oder von Erkrankungen, die mit einer tumoralen oder pathologischen Angiogenese verbunden sind.

4. Zusammensetzung nach einem der Ansprüche 1 bis 3 für eine topische äußerliche Anwendung oder für eine orale Verabreichung.

5. Zusammensetzung nach einem der vorangegangenen Ansprüche, **dadurch gekennzeichnet, dass** der peptidische Extrakt von Lupine wenigstens 50%, vorzugsweise wenigstens 70%, vorzugsweise wenigstens 80% Peptide umfasst.

6. Zusammensetzung nach einem der vorangegangenen Ansprüche, **dadurch gekennzeichnet, dass** die Peptide ein Molekulargewicht unter 10000 Dalton haben.

7. Zusammensetzung nach einem der vorangegangenen Ansprüche, **dadurch gekennzeichnet, dass** der peptidische Extrakt von Lupine die folgende Aminosäurezusammensetzung aufweist (als Gewichtsprozentsatz bezogen auf das Gesamtgewicht der Aminosäuren) :

| Aminosäuren | %/AS gesamt |
| --- | --- |
| ASP | 11,3 |
| GLU | 23,2 |
| SER | 5,1 |
| HIS | 1,7 |
| GLY | 3,4 |
| THR | 3,2 |
| ALA | 2,8 |
| ARG | 10,3 |
| TYR | 6,1 |
| CYS-CYS | 2,4 |
| VAL | 3,8 |
| MET | 0,2 |
| PHE | 7,0 |
| ILE | 3,3 |
| LEU | 7,9 |
| LYS | 3,7 |
| PRO | 4,4 |

8. Zusammensetzung nach einem der vorangegangenen Ansprüche, **dadurch gekennzeichnet, dass** der peptidische Extrakt von Lupine erhalten werden kann durch ein Verfahren, welches die folgenden Schritte umfasst:

- Herstellung eines Presskuchens von den Lipiden befreiter und zerkleinerter Lupine oder eines (lipidhaltigen) mikronisierten Lupinenmehls;
- Extraktion der löslichen Protein- und Ose-Fraktionen oder Ausfällung der Proteine am isoelektrischen Punkt,
- gegebenenfalls Abtrennung der Proteinfraktion,
- Hydrolyse der Proteinfraktion und gegebenenfalls Rückgewinnung, nach Filtration, des Proteinextrakts.

9. Zusammensetzung nach einem der vorangegangenen Ansprüche, **dadurch gekennzeichnet, dass** der peptidische Extrakt von Lupine durch Hydrolyse der Proteinfraktion der Lupine erhalten wird.

10. Zusammensetzung nach Anspruch 9, **dadurch gekennzeichnet, dass** die Hydrolyse der Proteinfraktion der Lupine eine enzymatische Hydrolyse ist.

11. Peptidischer Extrakt von Lupine, **dadurch gekennzeichnet, dass** er durch Hydrolyse der Proteinfraktion der Lupine erhalten wird.

12. Extrakt nach Anspruch 11, **dadurch gekennzeichnet, dass** die Hydrolyse der Proteinfraktion der Lupine eine enzymatische Hydrolyse ist.

13. Pharmazeutische, kosmetische oder nutrazeutische Zusammensetzung, umfassend einen Extrakt, wie in einem der Ansprüche 11 und 12 definiert.

14. Verwendung eines peptidischen Extrakts von Lupine für die Herstellung einer pharmazeutischen, kosmetischen oder nutrazeutischen Zusammensetzung, welche für die Behandlung von Menschen oder von Säugetieren, welche unter einer Erkrankung oder einem Zustand leiden, die bzw. der mit einer übermäßigen Zerstörung von Kollagen oder mit einer übermäßigen Zerstörung der Stütz-Makroproteine durch Metalloproteasen verbunden ist, bestimmt ist.

15. Verwendung nach Anspruch 14 für die Behandlung von Schädigungen der Haut, insbesondere von Schädigungen, die auf die intrinsische Alterung der Haut, auf die Alterung unter der Einwirkung der Sonnenstrahlung oder auf die schädlichen Wirkungen des Tabaks, der Verschmutzung oder von Stress zurückzuführen sind.

FIG. 1